# FASCICULE DE BREVET EUROPEEN

(11) **EP 0 562 915 B1**
(45) Date de publication et mention de la délivrance du brevet: **05.07.2000**
(21) Numéro de dépôt: 93400688.3
(22) Date de dépôt: 17.03.1993
(51) Int. Cl.: C07D 491/04, C07D 493/04, C07D 513/04, G03C 1/685

(54) **Chromènes hétérocycliques et leur utilisation dans le domaine de l'optique ophtalmique**
Heterozyklische Chromene und ihre Verwendung in der ophthalmischen Optik
Heterocyclic chromenes and their use in ophthalmic optics

(30) Priorité: 19.03.1992 FR 9203297
(43) Date de publication de la demande: 29.09.1993
(73) Titulaire: TRANSITIONS OPTICAL, INC., Pinellas Park, Florida 34660 (US)
(72) Inventeur: Guglielmetti, Robert, Résidence les Alisiers, F-13009 Marseille (FR); Pozzo, Jean-Luc, F-13008 Marseille (FR); Samat, André, F-13013 Marseille (FR)
(74) Mandataire: Catherine, Alain

(56) Documents cités:
- EP-A- 0 401 958
- WO-A-92/09593
- US-A- 3 567 605
- US-A- 5 066 818
- CHEMICAL ABSTRACTS, vol. 113, no. 9, 27 Août 1990, Columbus, Ohio, US; abstract no. 78167f, T. TANAKA ET AL. 'Naphthopyrans and analogs as photochromic agents and their preparation' page 762 ;
- BULLETIN DE LA SOCIETE CHIMIQUE DE FRANCE no. 8, 1967, PARIS FR pages 2728 - 2735 R. AZERAD 'No 490. Isomérisation acide des tocoquinones. II. Synthèse de dichromannes apparentés aux tocoquinones'
- CHEMICAL ABSTRACTS, vol. 115, no. 19, 11 Novembre 1991, Columbus, Ohio, US; abstract no. 207975k, SEIJI OKAZAKI ET AL 'Preparation of chromene compounds as photochromic substances' page 1021 ;

## Description

L'invention a pour objet des composés photochromiques, plus particulièrement des composés photochromiques comportant dans leur formule chimique un noyau de la famille des chromènes ou benzopyranes, leur utilisation dans le domaine de l'optique ophtalmique, en particulier dans et/ou sur des lentilles ophtalmiques.

Le photochromisme est un phénomène connu depuis de nombreuses années. On dit qu'un composé est photochromique lorsque ce composé, irradié par un faisceau lumineux, dont certaines longueurs d'onde se situent dans le domaine de l'ultraviolet, change de couleur et revient à sa couleur originelle dès que l'irradiation cesse.

Les applications de ce phénomène sont multiples, mais une des applications connues plus particulièrement intéressante concerne le domaine de l'optique ophtalmique.

De tels composés sont utilisables dans la fabrication de lentilles ou verres pour lunettes, en vue de filtrer les radiations lumineuses en fonction de leur intensité.

L'incorporation des composés photochromiques dans un matériau organique constituant une lentille ophtalmique, permet d'obtenir un verre dont le poids est considérablement réduit par rapport aux lentilles classiques en verre minéral qui comportent des halogénures d'argent à titre d'agent photochrome. Leur incorporation dans des matériaux organiques a toujours posé des difficultés techniques.

Toutefois, tout composé à propriété photochromique n'est pas forcément utilisable dans le domaine de l'optique ophtalmique. En effet, le composé photochromique doit répondre à un certain nombre de critères, dont entre autres :
- une forte colorabilité qui est la mesure de la capacité pour un composé photochromique de présenter une couleur intense après isomérisation;
- une coloration après absorption de la lumière rendant apte le composé photochromique, seul ou en combinaison avec d'autres composés photochromiques à être utilisé dans des verres ou lentilles ophtalmiques;
- une absence de coloration ou très faible coloration sous la forme initiale;
- une cinétique rapide de coloration ou de décoloration;
- un photochromisme se manifestant dans une plage de température la plus large possible, et en particulier de préférence entre 0 et 40°C.

Les composés photochromiques organiques connus et utilisés actuellement présentent généralement un photochromisme décroissant lorsque la température augmente, de sorte que le photochromisme est particulièrement marqué à des températures proches de 0°C, alors qu'il est beaucoup plus faible, voire inexistant, à des températures de l'ordre de 40°C qui sont des températures que peuvent atteindre les verres lors notamment de l'exposition au soleil.

Un autre problème rencontré pour les composés photochromiques de l'état de la technique est leur durée de vie. On constate en effet pour certains produits de l'état de la technique, une durée de vie relativement réduite. En effet, après un certain nombre de cycles de coloration et de décoloration, le composé photochromique se bloque généralement dans une forme ouverte et colorée et ne présente plus les propriétés photochromes réversibles.

De nombreux composés photochromiques de type chroménique ont été synthétisés par le Professeur HELLER dans, par exemple, la demande de brevet EP 246 114 décrivant une série de composés photochromiques dans lesquels un groupe spiroadamantane est introduit en position 2 du noyau beuzopyrane ou naphtopyrane, ou encore la demande de brevet WO 90/07507 où 2 groupes cyclopropyle sont attachés à la position 2 du composé cyclique de benzopyrane ou naphtopyrane. On peut encore citer, du même inventeur, la demande de brevet WO 91/00861 où un groupe norcamphore ou un groupe tricyclodécane est introduit en position 2 de composés photochromiques du même type.

On a déjà décrit dans le brevet US-3.567.605 des dérivés photochromiques de type benzopyrane et naphtopyrane substitués en position 2 du cyle pyrannique. Ces composés présentent cependant des constantes cinétiques de décoloration relativement faibles.

Le document Chem. Abs. 115, 1991, abs.no. 207975k décrit des composés photochromiques du type benzopyrano [6,7,8-i] quinolizine de structure générale :

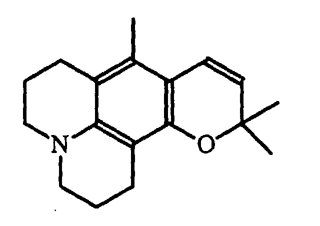

Le document Bulletin de la Société Chimique de France No. 8, 1967, 2728-2735 décrit des dichromanes apparentés aux tocoquinones.

Le document Chem. Abs. 113, 1990, abs.no. 78167F décrit des composés photochromiques du type naphtopyrane. Le composé exemplifié présente une couleur jaune par exposition à la lumière d'une lampe à vapeur de mercure.

Le document US-A-5 066 818 décrit des composés naphtopyranes photochromiques ayant au moins un groupe phényle substitué en ortho en position 3 du cycle pyranique.

Le document WO-A-92/09593 publié le 11.06.92 décrit des napthopyranes photochromiques substitués dans la partie naphtyle sur l'atome de carbone juxtaposé à l'oxygène du cycle pyranique. On connaît par ailleurs dans la demande EP-A-0401958 des dérivés photochromes présentant également des constantes de cinétique de décoloration faibles et moins bien adaptés à l'application envisagée.

La société demanderesse a découvert une nouvelle famille de benzopyranes présentant des propriétés photochromiques particulièrement intéressantes. Les composés conformes à l'invention présentent en effet une forte colorabilité en particulier dans le domaine rouge particulièrement utile pour l'optique ophtalmique, ces composés pouvant alors être utilisés avec des composés photochromiques donnant une couleur bleue, en vue d'obtenir une coloration finale naturelle lors de l'exposition à la lumière.

Les composés conformes à l'invention présentent par ailleurs une absence de coloration ou une très faible coloration à l'état initial et une cinétique rapide de coloration et de décoloration dans une plage de température très large, comprise en particulier entre 0 et 40°C.

La demanderesse a également constaté que ces composés avaient une durée de vie particulièrement longue.

Toutes ces propriétés font que ces composés photochromiques sont particulièrement intéressants dans leur utilisation dans l'optique ophtalmique et en particulier pour leur utilisation dans et/ou sur des lentilles ophtalmiques.

On appelle lentilles ophtalmiques au sens de l'invention, des verres de lunettes, en particulier des verres solaires, des lentilles de contact.

Un objet de l'invention est donc constitué par les composés photochromiques.

Un autre objet de l'invention est constitué par leur utilisation dans l'optique ophtalmique.

L'invention a également pour objet des compositions destinées à être utilisées pour le revêtement de lentilles ophtalmiques ou leur incorporation dans ces lentilles.

D'autres objets de l'invention apparaîtront à la lecture de la description et des exemples qui suivent.

Le composé photochromique, conforme à l'invention, est essentiellement caractérisé par le fait qu'il répond à la formule générale : 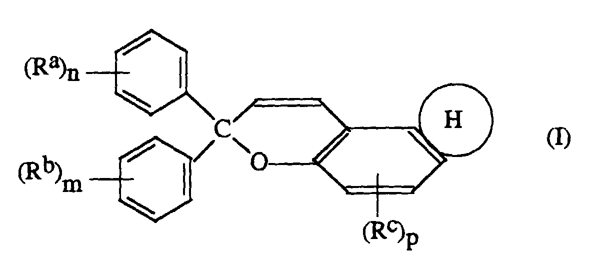 dans laquelle R^{a}, R^{b} et R^{c} désignent, indépendamment l'un de l'autre, un atome d'hydrogène; un groupement alkyle en C₁-C₆ ; un groupement phényle ; un groupement OR, SR, COR ou COOR, dans lesquels R désigne un atome d'hydrogène, un groupement alkyle en C₁-C₆ ou un groupement phényle ; un groupement amino de formule NR₁R₂ dans lequel R₁ et R₂ désignent, indépendamment l'un de l'autre, un atome d'hydrogène, un groupement alkyle en C₁-c₆, un groupement cycloalkyle ayant 3 à 7 atomes de carbone, un groupement phényle, un atome d'halogène; un groupement CF₃ ; un groupement NO₂, CN ou SCN; n et m désignent des nombres entiers de 1 à 5 suivant le nombre de substitutions sur le noyau et p peut être égal à 1 ou 2 suivant le nombre de substitutions sur le noyau ; les radicaux R^{a}, R^{b}, R^{c} peuvent avoir des significations différentes lorsque m, n et p sont supérieurs à 1 et suivant la position sur les noyaux; H est un hétérocycle choisi parmi les hétérocycles de formules : 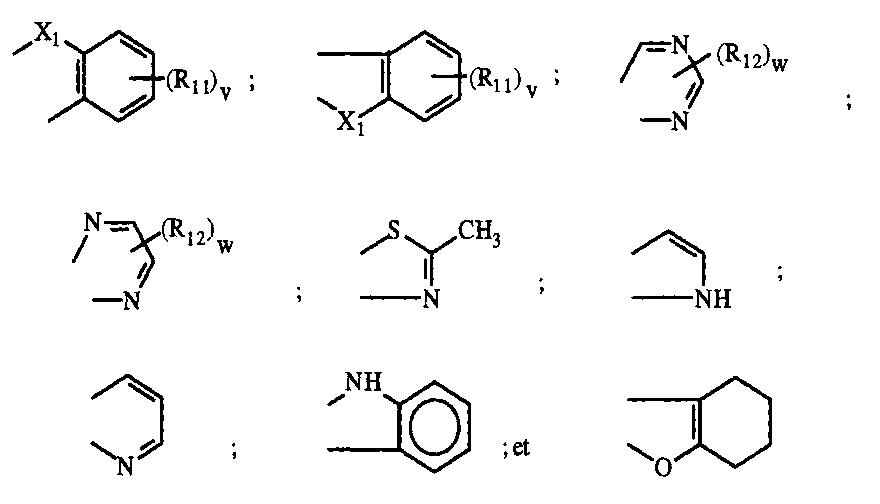 dans lesquelles R₁₁ et R₁₂ ont la même signification que l'un quelconque des groupements R^{a}, R^{b} et R^{c} définis ci-dessus et v est un entier de 0 à 4 et w un entier de 0 à 2, et X₁ représente l'oxygène ou le soufre, de préférence l'oxygène.

Dans la formule précitée, un groupement alkyle désigne de préférence un groupement ayant 1 à 6 atomes de carbone, un groupement cycloalkyle désigne de préférence un groupement ayant 3 à 7 atomes de carbone, le groupement aryle désigne de préférence un groupement phényle, et halogène désigne de préférence chlore, brome, fluor.

Les noyaux hétérocycliques plus particulièrement préférés sont choisis parmi les noyaux pyrimidinique, pyrazinique, furane éventuellement condensé avec un noyau aromatique pour former un cycle benzofurane, thiazole éventuellement substitué.

Les familles de composés plus particulièrement préférées répondent aux formules : 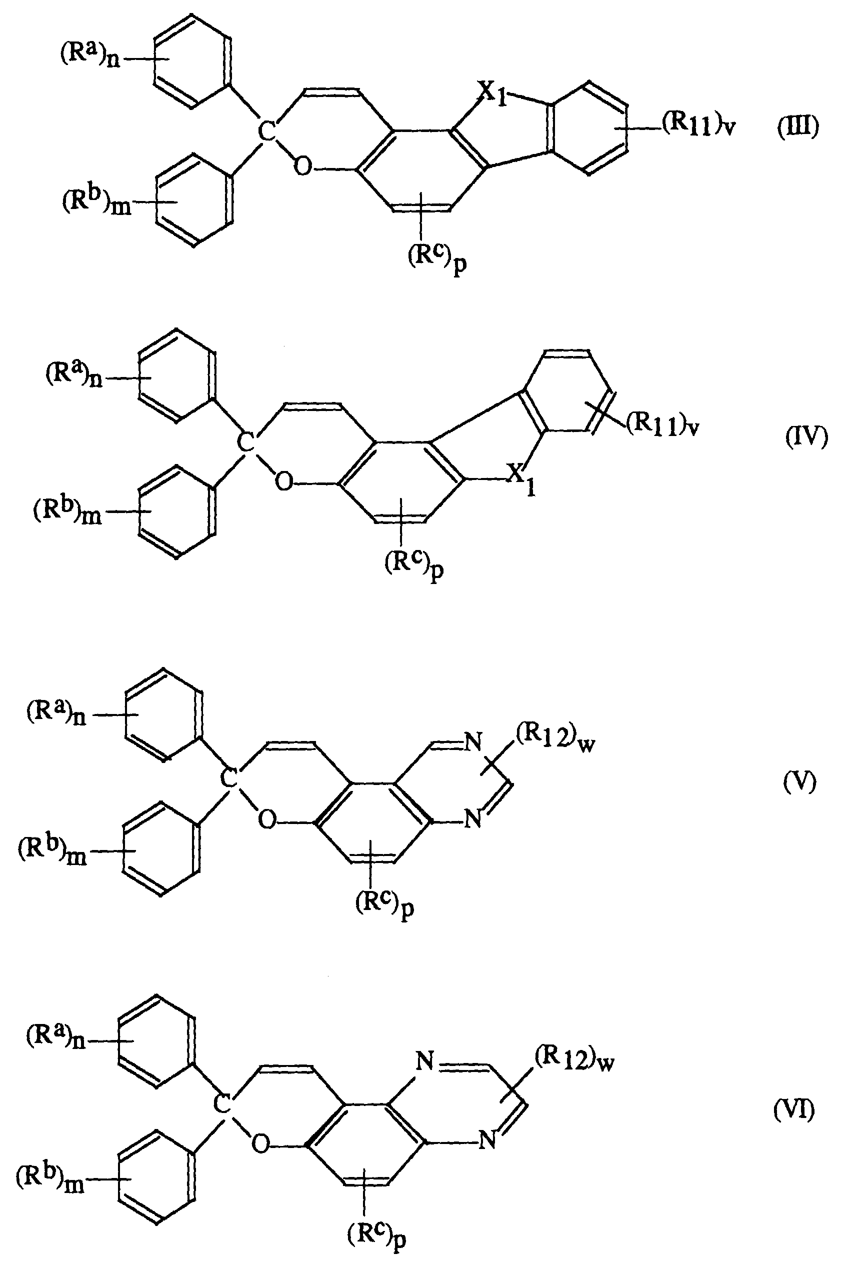 dans lesquelles R^{a} R^{b}, R^{c}, R₁₁, R₁₂, X₁ et n, m, p, v et w ont les significations indiquées ci-dessus.

Les composés conformes à l'invention peuvent être préparés selon le schéma réactionnel suivant : 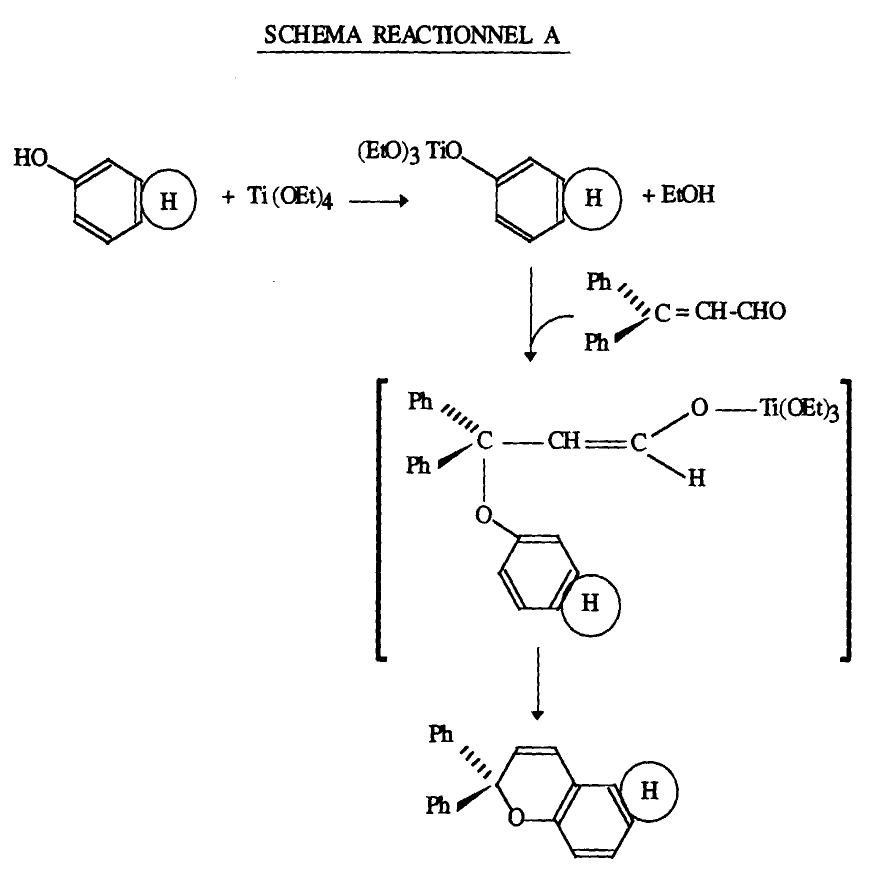 Dans ces formules, H désigne un groupement hétérocyclique ayant la signification indiquée ci-dessus. Les groupements phényle peuvent être substitués par un groupement R^{a} ou R^{b} tel que défini ci-dessus.

Les composés photochromiques conformes à l'invention peuvent être utilisés pour réaliser des lentilles ophtalmiques photochromiques.

Les composés conformes à l'invention peuvent être introduits dans une composition destinée à être appliquée sur ou être introduite dans un matériau polymère organique transparent pour obtenir un article transparent photochromique. Ils peuvent également être introduits dans des compositions solides telles que films plastique, plaques et lentilles pour réaliser des matériaux utilisables notamment comme lentilles ophtalmiques, lunettes de soleil, viseurs, optique de caméra et filtres.

Les compositions liquides qui constituent un objet de l'invention sont essentiellement caractérisées par le fait qu'elles contiennent sous forme dissoute ou dispersée les composés conformes à l'invention dans un milieu à base de solvants appropriés pour être appliqués ou introduits dans un matériau polymère transparent.

Des solvants plus particulièrement utilisables sont des solvants organiques choisis parmi le benzène, le toluène, le chloroforme, l'acétate d'éthyle, la méthyléthylcétone, l'acétone, l'alcool éthylique, l'alcool méthylique, l'acétonitrile, le tétrahydrofurane, le dioxane, l'éther méthylique d'éthylèneglycol, le diméthylformamide, le diméthylsulfoxyde, le méthylcellosolve, la morpholine, et l'éthylèneglycol.

Lorsque les composés conformes à l'invention sont dispersés, le milieu peut également contenir de l'eau.

Selon une autre forme de réalisation, les composés conformes à l'invention peuvent être introduits et de préférence dissous dans des solutions incolores ou transparentes préparées à partir de polymères, de copolymères ou de mélanges de polymères transparents dans un solvant organique approprié.

Les exemples de telles solutions sont entre autres des solutions de nitrocellulose dans l'acétonitrile, de polyvinylacétate dans l'acétone, de chlorure de polyvinyle dans la méthyléthylcétone, de polyméthylméthacrylate dans l'acétone, d'acétate de cellulose dans le diméthylformamide, de polyvinylpyrrolidone dans de l'acétonitrile, de polystyrène dans le benzène, d'éthylcellulose dans du chlorure de méthylène.

Ces compositions peuvent être appliquées sur des supports transparents tels qu'en térèphtalate de polyéthylèneglycol, de papier borylé, de triacétate de cellulose et séchées pour obtenir un matériau photochromique qui peut se colorer en présence d'une radiation ultraviolette, et qui retourne à l'état non coloré et transparent en l'absence de la source de radiation.

Les composes photochromiques de la présente invention ou les compositions les contenant définies ci-dessus peuvent être appliqués ou incorporés dans un matériau organique polymérisé transparent solide approprié pour des éléments ophtalmiques tels que des lentilles ophtalmiques ou des matériaux utiles pour être utilisés dans des lunettes de soleil, des viseurs, des optiques de caméras et des filtres.

A titre de matériaux solides transparents qui peuvent être utilisés pour réaliser des lentilles ophtalmiques conformes à l'invention, on peut citer les polymères de polyol(allylcarbonate), des polyacrylates, des poly(alkylacrylate) tels que des polyméthylméthacrylates, l'acétate de cellulose, le triacétate de cellulose, le propionate acétate de cellulose, le butyrate acétate de cellulose, le poly(vinylacétate), le poly(vinylalcool), les polyuréthanes, les polycarbonates, les polyéthylènetérèphtalates, les polystyrènes, les (polystyrène-méthylméthacrylate), les copolymères de styrène et d'acrylonitrile, les polyvinylbutyrates.

Les copolymères transparents ou des mélanges de polymères transparents sont également appropries pour réaliser de tels matériaux.

On peut citer à ce sujet les matériaux préparés à partir de polycarbonates tels que le poly(4,4'-dioxydiphénol-2,2 propane), le polyméthylméthacrylate, les polyol(allylcarbonate) tels qu'en particulier le diéthylèneglycol bis(allylcarbonate) et ses copolymères tels que par exemple avec l'acétate de vinyle. On peut citer en particulier les copolymères de diéthylèneglycol bis(allylcarbonate) et d'acétate de vinyle (80-90/10-20) et encore le copolymère de diéthylèneglycol bis(allylcarbonate) avec l'acétate de vinyle, l'acétate de cellulose et le propionate de cellulose, le butyrate de cellulose (80-85/15-20).

Les polyols(allylcarbonate) sont préparés en utilisant des allyl carbonates de polyols liquides aliphatiques ou aromatiques, linéaires ou ramifiés tels que les glycols aliphatiques de bis-allylcarbonate ou les alkylène bis(allylcarbonate). Parmi les polyols (allylcarbonate) qui peuvent être utilisés pour préparer les matériaux transparents solides utilisables conformément à l'invention, on peut citer l'éthyèneglycol bis(allylcarbonate), le diéthylèneglycol bis(2-méthallylcarbonate), le diéthylèneglycol bis(allylcarbonate), l'éthylèneglycol bis(2-chloro-allylcarbonate), le triéthylèneglycol bis(allylcarbonate), le 1,3-propanediol bis(allylcarbonate), le propylèneglycol bis(2-éthylallylcarbonate), le 1,3-butanediol bis(allylcarbonate), le 1,4-butanediol bis(2-bromoallylcarbonate), le dipropylèneglycol bis(allylcarbonate), le triméthylèneglycol bis(2-éthylallylcarbonate), le pentaméthylène glycol bis(allylcarbonate), l'isopropylène bisphénol bis (allyl carbonate). Le produit le plus important est constitué par le diéthylène glycol bis(allylcarbonate) encore connu sous la dénomination CR39.

La quantité de composés photochromiques à utiliser conformément à l'invention, soit dans la composition, soit au moment de son introduction dans le support solide, n'est pas critique et dépend généralement de l'intensité de la couleur que la composition peut conférer au matériau après exposition aux radiations. D'une manière générale, plus on ajoute de composés photochromiques, plus la coloration sous irradiation sera importante.

Conformément à l'invention, on utilise une quantité suffisante pour conférer au matériau traité la propriété de changer de couleur au moment de l'exposition à la radiation. Cette quantité de composés photochromiques est généralement comprise entre 0,01 et 20% en poids et de préférence entre 0,05 et 10% en poids par rapport au poids total du matériau optique ou de la composition.

Les composés photochromiques conformes à l'invention peuvent également être introduits dans un support temporaire (tel qu'un vernis formant un revêtement sur un substrat) de transfert et être transférés ensuite thermiquement dans le substrat comme décrit en particulier dans le brevet US-4.286.957 ou US-4.880.667.

Ces composés peuvent être utilisés avec d'autres composés photochromiques, tels que des composés photochromiques donnant lieu à des colorations différentes telles que bleu, verte, connus dans l'état de la technique. C'est ainsi qu'on peut utiliser des spiro(indoline-oxazines) bien connus dans l'état de la technique.

Une fois appliqués sur des matériaux ophtalmiques ou introduits dans de tels matériaux, on constate après exposition aux irradiations UV, l'apparition d'une coloration et le retour à la couleur ou à la transparence originelle lorsqu'on interrompt l'exposition aux radiations UV.

Les composés conformes à l'invention présentent l'intérêt de permettre ce changement de coloration un grand nombre de fois et ceci à des températures très variables comprises entre 0 et 40°C.

Les exemples suivants sont destinés à illustrer l'invention sans pour autant présenter un caractère limitatif.

### EXEMPLE 1

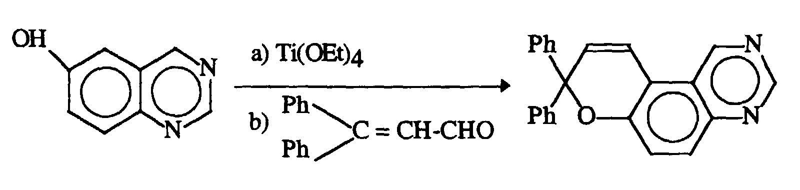

2,09 g de 6-hydroxyquinazoline (1,43.10⁻² mole) sont dissous dans 10 ml de toluène anhydre et placés sous atmosphère inerte.

Une solution toluénique stoechiométrique d'orthotitanate est additionnée. On porte ensuite le mélange réactionnel au reflux pendant 1 heure. On poursuit ensuite le chauffage afin d'éliminer l'éthanol ainsi formé. Après retour à température ambiante, on additionne 1,46 g (7,01.10⁻³ mole) de β-phénylcinnamaldéhyde. On poursuit le reflux ensuite pendant 2 heures sous atmosphère inerte. Après retour à température ambiante, le milieu réactionnel est extrait à l'aide d'une solution 2M de chlorure d'ammonium, puis trois fois à l'aide d'une solution 2M de soude.

On sèche sur MgSO₄. On chasse le solvant à l'évaporateur rotatif. On additionne 50 ml d'hexane. On recueille le précipité que l'on recristallise dans le cyclohexane (solide blanc).
Point de fusion = 162°C.
Rendement = 43%.

Quand une solution toluénique du composé de l'exemple 1 est irradiée par le rayonnement UV, la solution devient rouge (438 nm), quand l'irradiation ultraviolette est coupée, la solution redevient incolore.

### EXEMPLE 2

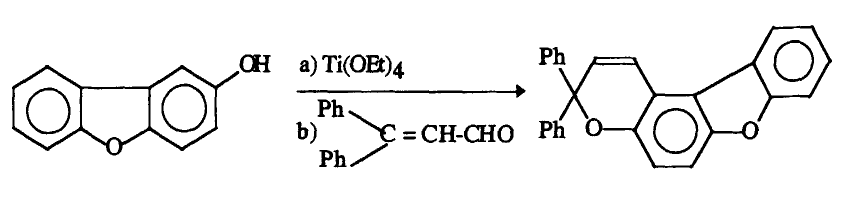

a) Le 2-hydroxydibenzofurane (1,842 g, 10⁻² mole) est dissous dans le toluène anhydre. On additionne une solution toluénique stoechiométrique d'orthotitanate. On porte le mélange réactionnel au reflux pendant 30 minutes puis on distille l'éthanol ainsi formé.
b) Après retour à température ambiante, on additionne lentement une solution toluénique de β-phénylcinnamaldéhyde (1,041 g, 5.10⁻³ mole). On poursuit le reflux sous atmosphère inerte pendant 1 h 30.

Après retour à température ambiante, on chasse le solvant à l'évaporateur rotatif. On additionne alors du dichlorométhane ainsi qu'une solution 2N de soude.

On extrait en continu pendant 24 heures. On sèche sur MgSO₄. On chasse le solvant à l'évaporateur rotatif.

Le chromène obtenu est purifié par chromatographie "flash" (100% pentane). On recristallise dans le cyclohexane (solide jaune).
Point de fusion = 134°C.
Rendement = 40%.

Quand une solution toluénique de l'exemple 2 est irradiée par le rayonnement UV, la solution devient rouge (505 nm), quand l'irradiation ultraviolette est coupée, la solution redevient incolore.

### EXEMPLE 3

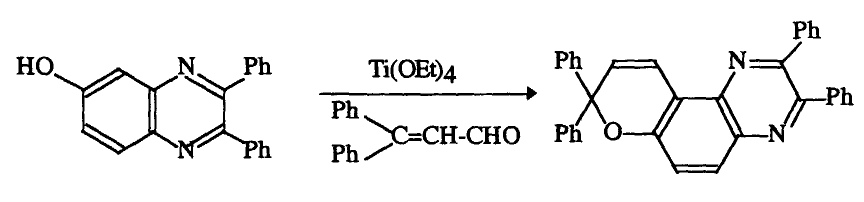

### Etape 1 : Synthèse de la 6-hydroxy 2,3-diphénylquinoxaline.

### a) 6-méthoxy 2,3-diphénylquinoxaline

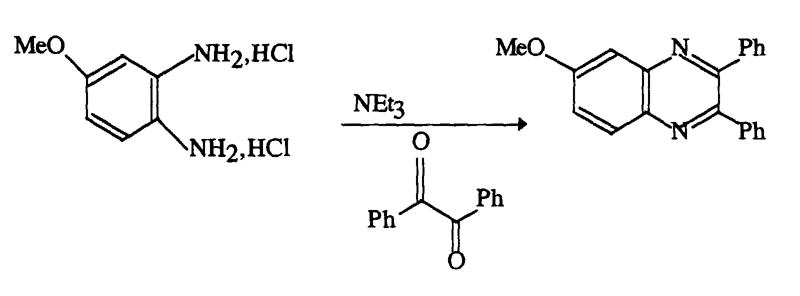

2 g de dichlorhydrate de 4-méthoxy 1,2-phénylènediamine (1,15. 10⁻² mole) sont dissous dans 20 ml d'éthanol anhydre sous atmosphère inerte.

On additionne une solution éthanolique stoechiométrique de triéthylamine. On poursuit l'agitation à température ambiante pendant 30 minutes. On additionne une solution éthanolique de benzil (2,46 g 1,17.10⁻² mole). On porte au reflux pendant 4 heures. On récupère par filtration le précipité formé. On sèche sous vide afin d'obtenir 2,4 g du composé désiré.
Point de fusion = 152°C
Rendement = 70%.

### b) 6-hydroxy 2,3-diphénylquinoxaline

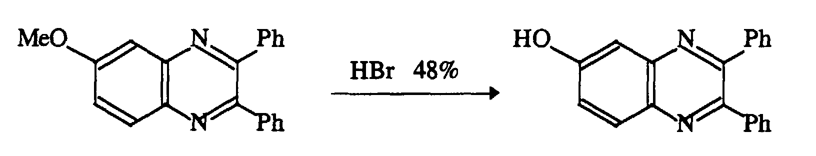

Un mélange de 6-méthoxy 2,3-diphénylquinoxaline (1 g : 3,2.10⁻³ mole) et de 10 ml d'HBr (48%) est chauffé à 120°C pendant 9 heures. Après retour à température ambiante, la solution est neutralisée (pH : 7,5) à l'aide d'une solution 1M de NH₄OH.

On récupère par filtration le précipité formé que l'on purifie par chromatographie "flash" (99%, CHCl₃, 1%, CH₃OH). On chasse le solvant à l'évaporateur rotatif.
Point de fusion = 251°C
Rendement = 43%.

### Etape 2 :

a) La 6-hydroxy 2,3-diphénylquinoxaline (1 g, 3,37.10⁻³ mole) est dissoute dans le toluène anhydre. On additionne une solution toluénique stoechiométrique d'orthotitanate. On porte le mélange réactionnel au reflux pendant 30 minutes puis on distille l'éthanol ainsi formé.
b) Après retour à température ambiante, on additionne lentement une solution toluénique de β-phénylcinnamaldéhyde (0,33 g, 1,58.10⁻³ mole). On porte à reflux sous atmosphère inerte pendant 2 heures.

Après retour à température ambiante, le milieu réactionnel est extrait à l'aide d'une solution 2M de chlorure d'ammonium, puis trois fois à l'aide d'une solution 2M de soude. On sèche sur MgSO₄. On chasse le solvant à l'évaporateur rotatif. On additionne 25 ml de cyclohexane. On recueille un précipité que l'on purifie par chromatographie (100% éther). On recristallise dans le cyclohexane (solide blanc).
Point de fusion = 159°C
Rendement = 10%

### EXEMPLE 4

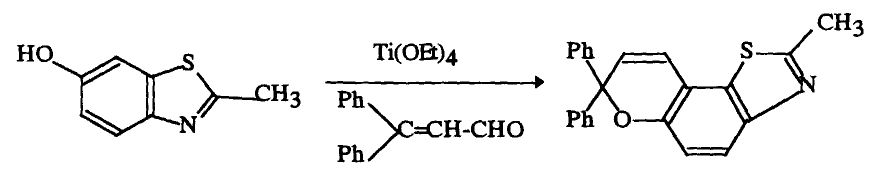

### Etape 1 : Synthèse du 6-hydroxy 2-méthylbenzothiazole

On mélange 1 g de 6-méthoxy 2-méthylbenzotbiazole commercial (5,5 x 10⁻³ mole) à 0,9 g d'acide bromhydrique azéotropique à 48% (1,1 x 10⁻² mole). La réaction s'effectue en tube scellé à 125°C durant 6 heures.

Après neutralisation de la solution avec de l'ammoniaque 3N (pH=7), le composé hydroxylé est extrait au chloroforme. Le rendement est quantitatif. (Synthèse décrite dans le brevet français FR-2.647.790).
Point de fusion = 147°C.

### Etape 2 :

0,965 g de 6-hydroxy 2-méthylbenzothiazole (5,83.10⁻³ mole) sont dissous dans 10 ml de toluène anhydre et placés sous atmosphère inerte.

Une solution toluénique stoechiométrique d'orthotitanate est additionnée. On porte ensuite le mélange réactionnel au reflux pendant 30 minutes. On poursuit ensuite le chauffage afin d'éliminer l'éthanol ainsi formé. Après retour à température ambiante, on additionne 0,585 g (2,8.10⁻³ mole) de β-phénylcinnamaldéhyde. On poursuit le reflux ensuite pendant 1 h 30 sous atmosphère inerte. Après retour à température ambiante, le milieu réactionnel est extrait à l'aide d'une solution 2M de chlorure d'ammonium, puis trois fois à l'aide d'une solution 2M de soude.

On sèche sur MgSO₄. On chasse le solvant à l'évaporateur rotatif. On additionne 50 ml d'hexane. On recueille le précipité que l'on recristallise dans le cyclohexane (solide jaune).
Point de fusion = 215°C Rendement = 35%

**Tableau 1**

| **Paramètres spectro-cinétiques dans le toluène à 25°C à une concentration de 2,5 x 10**^{**-5**} **M** | | | | |
|---|---|---|---|---|
| | Couleur de la photo-merocyanine | λₘₐₓ (nm) | Constante cinétique de décoloration thermique K en s⁻¹ | Aₒ colorabilité |
| Exemples | | | | |
| 1 | Rouge | 438 | 0,47 | 1,03 |
| 2 | Rouge | 505 | 0,23 | 0,41 |
| 3 | Rouge | 471 | 0,1 | 0,93 |
| 4 | Rouge | 430-508 | 0,25 | 0,14 |

### EXEMPLE 5

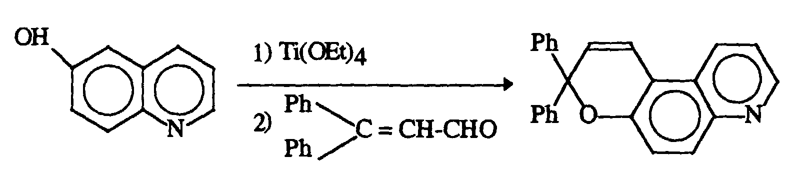

a) 3 g de 6-quinolinol (2,07 x 10⁻² mole) sont dissous dans du toluène anhydre. On additionne une solution stoechiométrique d'orthotitanate (4,722 g, soit 2,07 x 10⁻² mole d'orthotitanate). On porte le mélange au reflux pendant 1 heure, puis on distille l'éthanol formé.
b) On additionne ensuite lentement une solution toluénique de β-phénylcinnamaldéhyde (2,047 g, 9,83 x 10⁻³ mole).

Après retour à température ambiante, on lave avec une solution de chlorure d'ammonium, puis une solution de soude 2M.

La phase aqueuse est séchée sur MgSO₄, le solvant est chassé sous pression réduite.

On purifie par chromatographie flash (80% pentane, 20% Et₂O). On recristallise dans un mélange xylène-heptane.
Point de fusion = 222°C.
Rendement = 62%.

### EXEMPLE 6

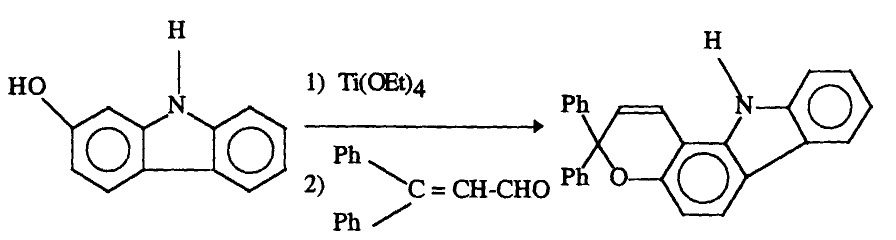

1,832 g (10 mmoles) de 2-hydroxycarbazole sont dissous dans 50 ml de toluène anhydre et placés sous atmosphère inerte.

Une solution toluénique (10 ml) de 2,28 g d'orthotitanate (10 mmoles) est additionnée progressivement.

Le mélange réactionnel est porté au reflux du toluène pendant 40 minutes et on distille simultanément l'éthanol.

On laisse revenir à température ambiante avant d'additionner une solution toluénique (35 ml) de 1 g de (β-phénylcinnamaldéhyde (4,8 mmoles). En fin d'addition, on porte le mélange au reflux pendant 2 heures. Le solvant est chassé sous pression réduite.

On rince avec une solution de NH₄Cl 2M, puis avec une solution de NaOH 2M. Il apparaît alors une importante émulsion, reprise au chloroforme. On laisse décanter.

La phase aqueuse est filtrée après avoir ajusté le pH à 8,5. On récupère l'alcool.

Le solvant de la phase organique est chassé sous pression réduite. Le résidu précipite dans l'hexane. On le filtre. Le produit obtenu est purifié par chromatographie flash avec comme éluant un mélange pentane/éther (75/25).
Masse obtenue = 0,3 g
Poids moléculaire (g. mol.) = 375,5
Point de fusion = 182°C
Rendement = 17%

**Tableau 2**

| **Paramètres spectro-cinétiques dans le toluène à 25°C à une concentration de 2,5 x 10**^{**-5**} **M** | | | | |
|---|---|---|---|---|
| | Couleur de la photo-merocyanine | λₘₐₓ (nm) | Constante cinétique de décoloration thermique K en s⁻¹ | Aₒ colorabilité |
| Exemples | | | | |
| 5 | Orange | 436 | 0,13 | 0,61 |
| 6 | Rouge | 436 | 0,15 | 0,99 |

### EXEMPLE 7

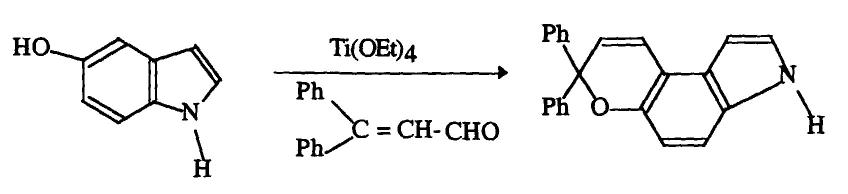

O,77 g de 5-hydroxyindole (5,77.10⁻³ mole) sont dissous dans 10 ml de toluène anhydre et placés sous atmosphère inerte.

Une solution toluénique stoechiométrique d'orthotitanate est additionnée. On porte ensuite le mélange réactionnel au reflux pendant 40 minutes. On poursuit ensuite le chauffage afin d'éliminer l'éthanol ainsi formé. Après retour à température ambiante, on additionne 0,55g (2,66.10⁻³ mole) de β-phénylcinnamaldéhyde. On poursuit le reflux ensuite pendant 1 heure 30 minutes sous atmosphère inerte. Après retour à température ambiante, le milieu réactionnel est extrait à l'aide d'une solution 2M de chlorure d'ammonium, puis trois fois à l'aide d'une solution 2M de soude.

On sèche sur MgSO₄. On chasse le solvant à l'évaporateur rotatif. On purifie par chromatographie sur silice (100% toluène). On recueille une huile rouge que l'on cristallise dans le pentane.
Point de fusion = 143°C
Rendement = 33%.

### EXEMPLE 8

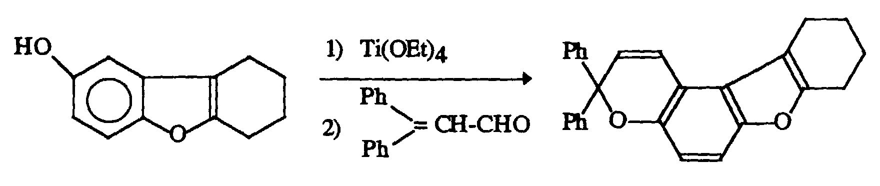

2,16 g (1,146 x 10-2 mole) de 2-hydroxy 5,6,7,8-tétrahydrodibenzofurane sont dissous dans 10 cm³ de toluène, puis on additionne une solution stoechiométrique d'orthotitanate (2,614 g, 1,146 x 10⁻² mole).

Le mélange est chauffé au reflux 1 heure, puis on distille l'éthanol formé.

Le mélange est ramené à température ambiante.

On ajoute ensuite une solution toluénique renfermant 1,313 g, soit 6,3 x 10⁻³ mole de β-phénylcinnamaldéhyde.

On chauffe à relfux pendant 3 h 30 minutes.

Après retour à température ambiante, le milieu réactionnel est extrait à la soude 2M.

On sèche avec MgSO₄, on chasse le solvant.

On purifie par chromatographie flash (85% hexane, 19% Et₂O). On recristallise dans un mélange benzène-heptane.
Point de fusion = 187°C
Rendement = 10%.

## Revendications

1. Composé photochromique, caractérisé par le fait qu'il répond à la formule générale : 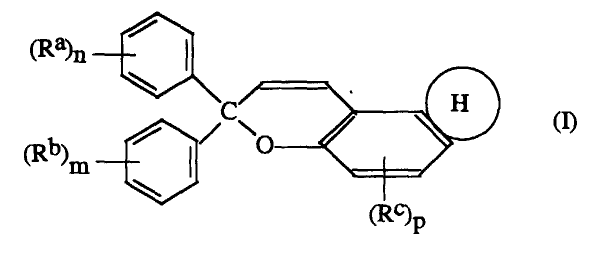 dans laquelle R^{a}, R^{b} et R^{c} désignent, indépendamment l'un de l'autre, un atome d'hydrogène, un groupement alkyle en C₁-C₆, un groupement phényle, un groupement OR, SR, COR ou COOR, dans lesquels R désigne un atome d'hydrogène, un groupement alkyle en C₁-C₆ ou un groupement phényle, un groupement amino de formule NR₁R₂ dans lequel R₁ et R₂ désignent, indépendamment l'un de l'autre, un atome d'hydrogène, un groupement alkyle en C₁-C₆, un groupement cycloalkyle ayant 3 à 7 atomes de carbone, un groupement phényle, un atome d'halogène, un groupement CF₃; un groupement NO₂, CN ou SCN; n et m désignent des nombres entiers de 1 à 5 suivant le nombre de substitutions sur le noyau et p peut être égal à 1 ou 2 suivant le nombre de substitutions sur le noyau; les groupements R^{a}, R^{b}, R^{c} peuvent avoir des significations différentes lorsque m, n et p sont supérieurs à 1 et suivant la position sur les noyaux; H est un hétérocycle aromatique ayant 4 à 7 chaînons comportant un ou plusieurs hétéroatomes choisis parmi les hétérocycliques de formules: 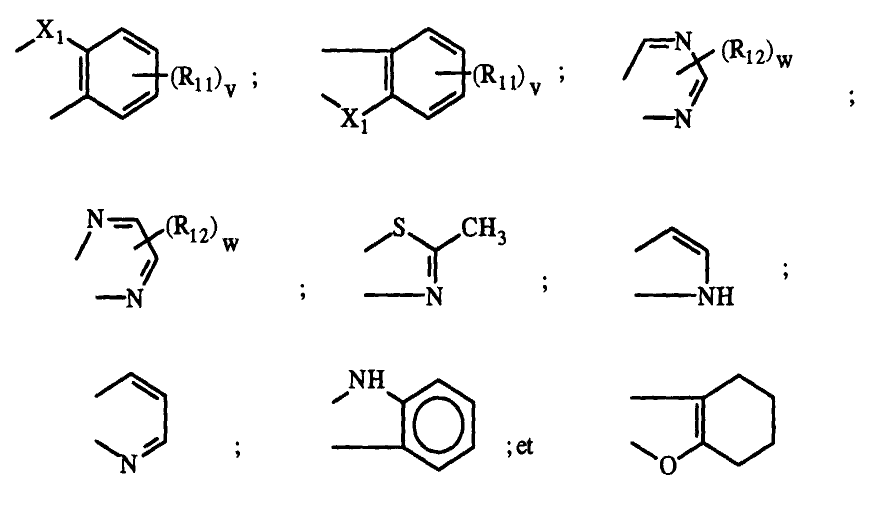 dans lesquelles R₁₁ et R₁₂ ont la même signification que l'un quelconque des groupements R^{a}, R^{b} et R^{c} définis ci-dessus et v est un entier de 0 à 4 et w un entier de 0 à 2, et X₁ représente l'oxygène ou le soufre, de préférence l'oxygène.

2. Composé selon la revendication 1, caractérisé en ce qu'il est choisi parmi les composés de formules :

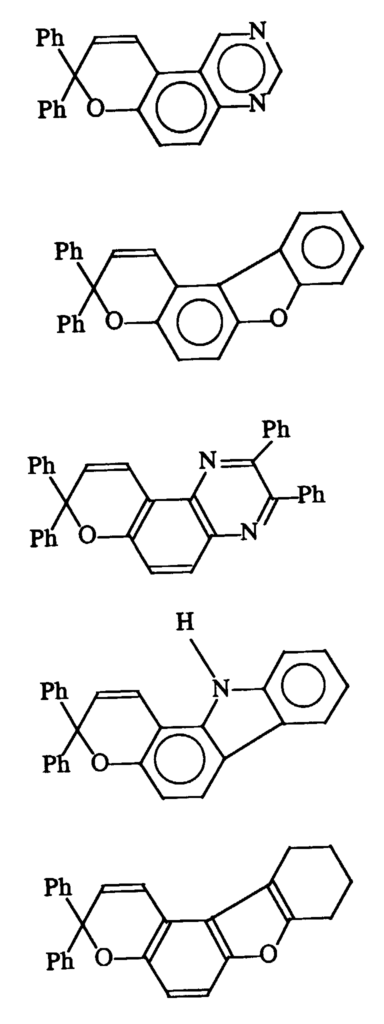

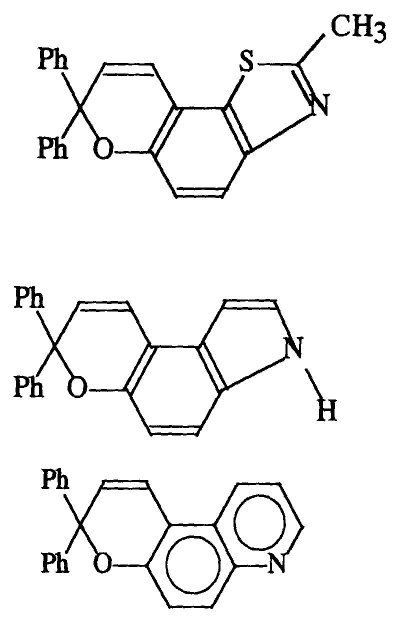

3. Utilisation du composé selon la revendication 1 ou 2, comme composé photochromique dans l'optique ophtalmique.

4. Composition destinée à être appliquée sur ou introduite dans un matériau polymère organique transparent, caractérisée par le fait qu'elle confient au moins un composé photochromique tel que défini dans la revendication 1 ou 2, dans des quantités suffisantes pour permettre au matériau exposé à une radiation ultraviolette de changer de couleur.

5. Composition selon la revendication 4, caractérisée par le fait que la composition est sous forme liquide contenant sous forme dissoute ou dispersée, les composés photochromiques selon la revendication 1 ou 2, dans un milieu à base de solvants appropriés pour être appliqués ou introduits dans un matériau polymère transparent.

6. Composition destinée à être appliquée sur ou introduite dans un matériau polymère organique transparent, caractérisée par le fait qu'elle est constituée par une solution incolore ou transparente à base de polymères, de copolymères ou de mélange de polymères transparents dans un solvant organique approprié, contenant au moins un composé photochromique tel que défini dans la revendication 1 ou 2, dans des quantités suffisantes pour permettre au matériau exposé à une radiation ultraviolette de changer de couleur.

7. Matériau solide transparent approprié pour réaliser des lentilles ophtalmiques, caractérisé par le fait qu'il comporte sur la surface et/ou à l'intérieur au moins un composé photochromique tel que défini dans la revendication 1 ou 2, dans des quantités suffisantes pour permettre au matériau exposé à une radiation ultraviolette de changer de couleur.

8. Matériau solide transparent selon la revendication 7, caractérisé par le fait qu'il confient 0,01 à 20% en poids de composés photochromiques.

9. Composition ou matériau solide transparent, selon l'une quelconque des revendications 4 à 8, caractérisée par le fait que le composé photochromique tel que défini dans la revendication 1 ou 2, est utilisé conjointement avec d'autres composés photochromiques donnant lieu à des colorations différentes.

10. Vernis de transfert, caractérisé par le fait qu'il contient au moins un composé tel que défini dans la revendication 1 ou 2.

## Claims

1. Photochromic compound, characterized in that it corresponds to the general formula: 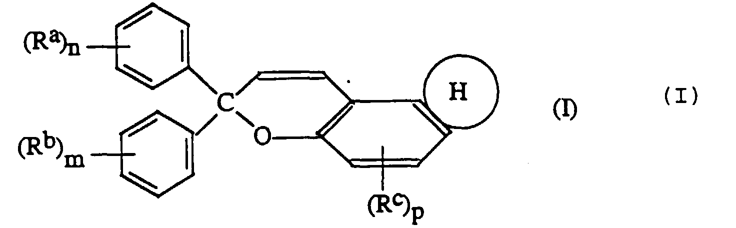 in which R^{a}, R^{b} and R^{c} denote, independently of each other, a hydrogen atom, a C₁-C₆ alkyl group, a phenyl group, an OR, SR, COR or COOR group, in which R denotes a hydrogen atom, a C₁-C₆ alkyl group or a phenyl group, an amino group of formula NR₁R₂ in which R₁ and R₂ denote, independently of each other, a hydrogen atom, a C₁-C₆ alkyl group, a cycloalkyl group having 3 to 7 carbon atoms, a phenyl group, a halogen atom, a CF₃ group; an NO₂, CN or SCN group; n and in denote integers from 1 to 5 depending on the number of substitutions on the nucleus and p may be equal to 1 or 2 depending on the number of substitutions on the nucleus; the groups R^{a}, R^{b} and R^{c} may have different meanings when m, n and p are greater than 1 and depending on the position on the nuclei; H is an aromatic heterocycle having 4 to 7 members containing one or more heteroatoms chosen from the heterocycles of formulae 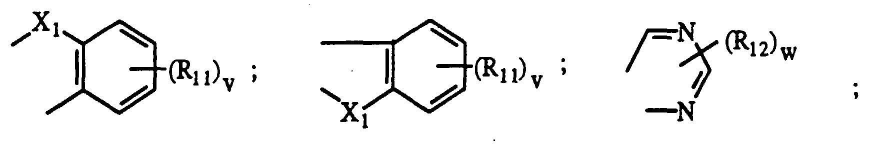 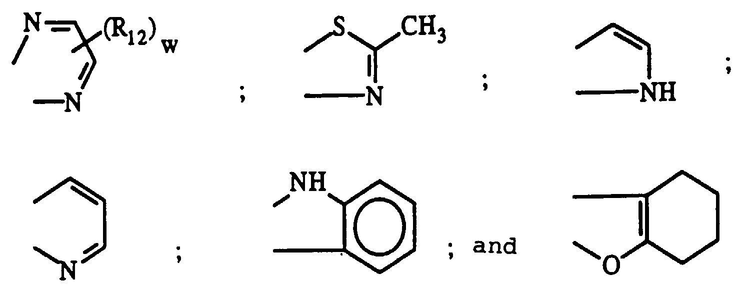 in which R₁₁ and R₁₂ have the same meaning as any one of the groups R^{a}, R^{b} and R^{c} defined above and v is an integer from 0 to 4 and w an integer from 0 to 2, and X₁ represents oxygen or sulphur, preferably oxygen.

2. Compound according to Claim 1, characterized in that it is chosen from the compounds of formulae:

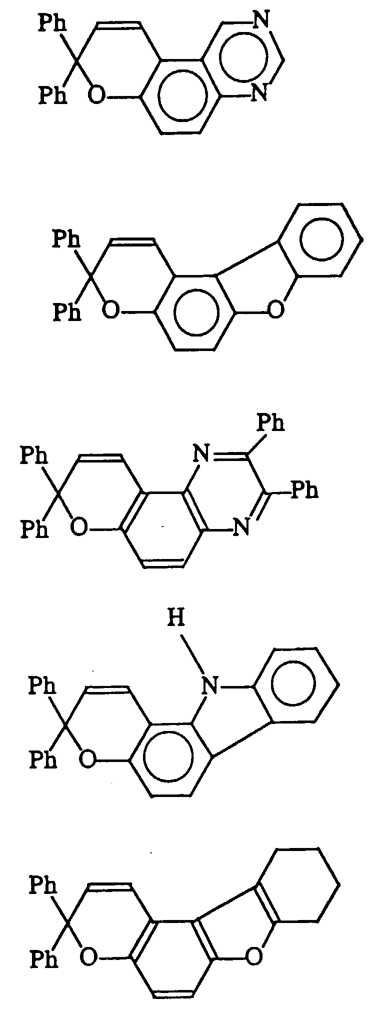

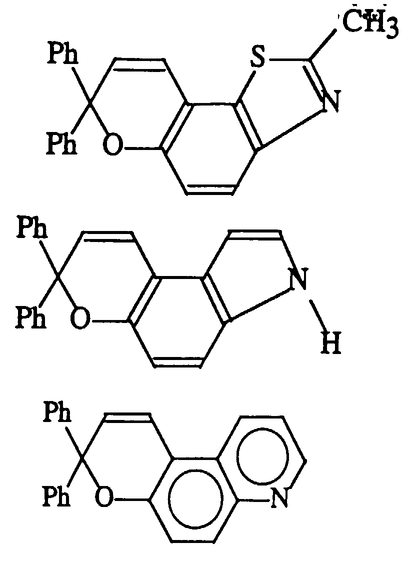

3. Use of the compound according to Claim 1 or 2, as a photochromic compound in ophthalmic optics.

4. Composition intended to be applied to or introduced into a transparent organic polymer material, characterized in that it contains at least one photochromic compound as defined in Claim 1 or 2, in amounts sufficient to enable a material exposed to ultraviolet radiation to change colour.

5. Composition according to Claim 4, characterized in that the composition is in liquid form containing in dissolved or dispersed form, the photochromic compounds according to Claim 1 or 2, in a medium based on solvents suitable tor applying to or introducing into a transparent polymer material.

6. Composition intended to be applied on or introduced into a transparent organic polymer material, characterized in that it consists of a colourless or transparent solution based on polymers, copolymers or a mixture of polymers which are transparent in a suitable organic solvent, containing at least one photochromic compound as defined in Claim 1 or 2, in amounts sufficient to enable the material to change colour when exposed to ultraviolet radiation.

7. Transparent solid material suitable for producing ophthalmic lenses, characterized in that it has at the surface and/or on the inside, at least one photochromic compound as defined in Claim 1 or 2, in amounts sufficient to enable the material to change colour when exposed to ultraviolet radiation.

8. Transparent solid material according to Claim 7, characterized in that it contains 0.01 to 20% by weight of photochromic compounds.

9. Transparent solid composition or material according to any one of Claims 4 to 8, characterized in that the photochromic compound as defined in Claim 1 or 2, is used together with other photochromic compounds giving rise to different colorations.

10. Transfer varnish, characterized in that it contains at least one compound as defined in Claim 1 or 2.

## Patentansprüche

1. Photochrome Verbindung, dadurch gekennzeichnet, daß sie der allgemeinen Formel 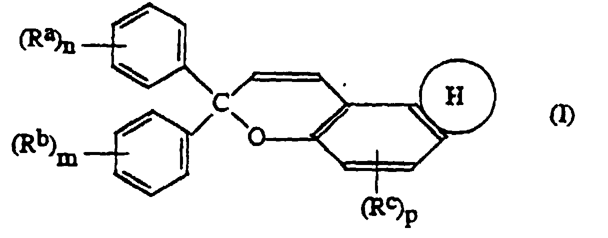 entspricht, in der R^{a}, R^{b} und R^{c} unabhängig voneinander für ein Wasserstoffatom, eine C₁-C₆-Alkylgruppe, eine Phenylgruppe, eine Gruppe OR, SR, COR oder COOR, worin R für ein Wasserstoffatom, eine C₁-C₆-Alkylgruppe oder eine Phenylgruppe steht, eine Aminogruppe der Formel NR₁R₂, worin R₁ und R₂ unabhängig voneinander für ein Wasserstoffatom, eine C₁-C₆-Alkylgruppe, eine Cycloalkylgruppe mit 3 bis 7 Kohlenstoffatomen, eine Phenylgruppe, ein Halogenatom, eine CF₃-Gruppe oder eine NO₂-, CN- oder SCN-Gruppe stehen, n und m je nach der Zahl der Substitutionen am Kern für ganze Zahlen von 1 bis 5 stehen und p je nach der Zahl der Substitutionen am Kern gleich 1 oder 2 sein kann, stehen, wobei die Gruppen R^{a}, R^{b} und R^{c} für den Fall, daß m, n und p größer als 1 sind, und je nach der Position an den Kernen verschiedene Bedeutungen haben können, und H für einen 4- bis 7-gliedrigen aromatischen Heterocyclus mit einem oder mehreren Heteroatomen, ausgewählt unter den Heterocyclen der Formeln: 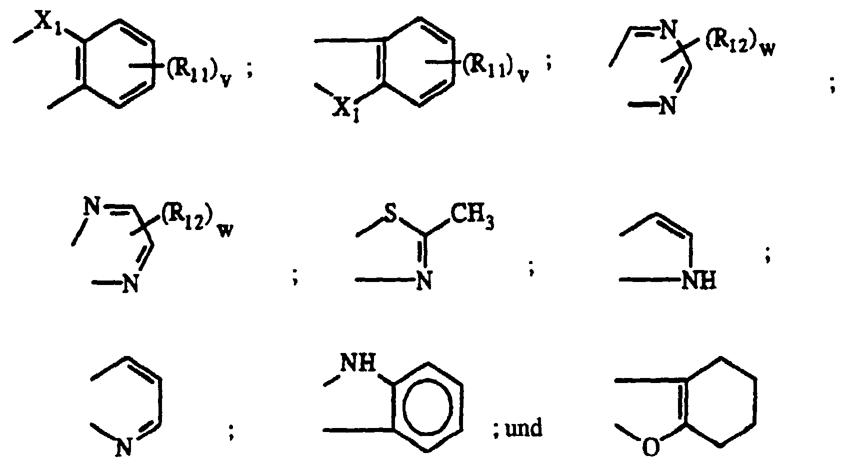 worin R₁₁ und R₁₂ die gleiche Bedeutung wie eine der oben definierten Gruppen R^{a}, R^{b} und R^{c} haben, v eine ganze Zahl von 0 bis 4, w eine ganze Zahl von 0 bis 2 und X₁ Sauerstoff oder Schwefel, vorzugsweise Sauerstoff, bedeutet, steht.

2. Verbindung nach Anspruch 1, dadurch gekennzeichnet, daß sie unter den Verbindungen der Formeln: 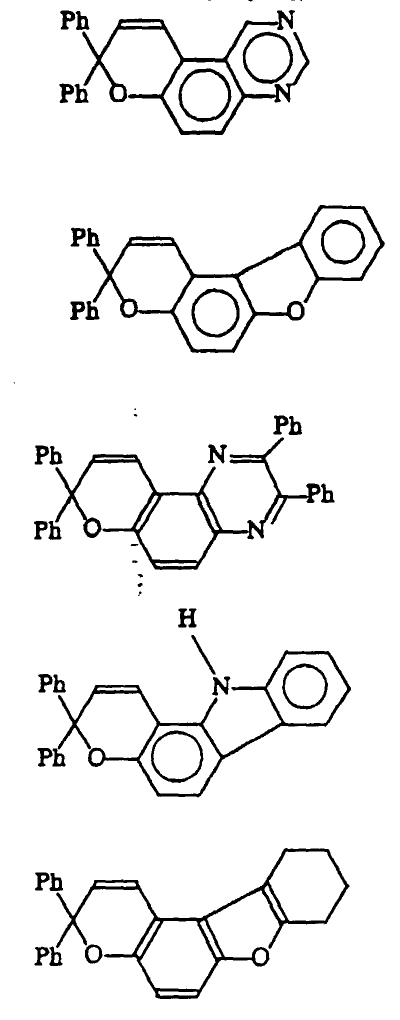 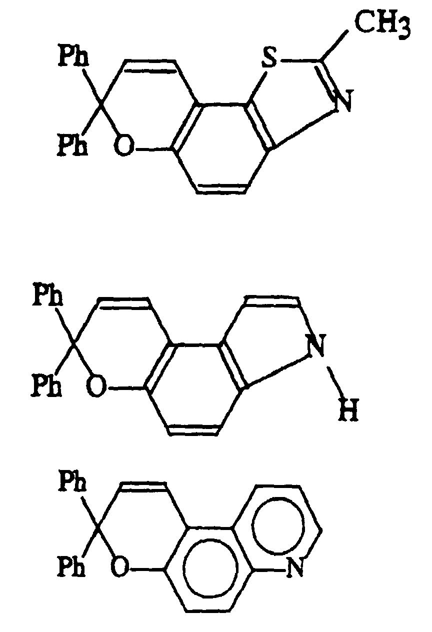 ausgewählt ist.

3. Verwendung einer Verbindung nach Anspruch 1 oder 2 als photochrome Verbindung in der ophthalmischen Optik.

4. Zusammensetzung zum Aufbringen auf oder Einarbeiten in ein transparentes organisches Polymermaterial, dadurch gekennzeichnet, daß sie mindestens eine photochrome Verbindung gemäß Anspruch 1 oder 2 in Mengen enthält, die dazu ausreichen, daß das Material bei Einwirkung von ultravioletter Strahlung die Farbe wechselt.

5. Zusammensetzung nach Anspruch 4, dadurch gekennzeichnet, daß sie in flüssiger Form vorliegt, die die photochromen Verbindungen gemäß Anspruch 1 oder 2 in einem Medium auf Basis von Lösungsmitteln, die sich für das Aufbringen auf oder Einarbeiten in ein transparentes polymeres Material eignen, in gelöster oder dispergierter Form enthält.

6. Zusammensetzung zum Aufbringen auf oder Einarbeiten in ein transparentes organisches Polymermaterial, dadurch gekennzeichnet, daß sie aus einer farblosen oder transparenten Lösung auf Basis von Polymeren, Copolymeren oder eines Gemischs aus transparenten Polymeren in einem geeigneten organischen Lösungsmittel besteht und mindestens eine photochrome Verbindung gemäß Anspruch 1 oder 2 in Mengen enthält, die dazu ausreichen, daß das Material bei Einwirkung von ultravioletter Strahlung die Farbe wechselt.

7. Transparentes festes Material für die Herstellung von ophthalmischen Linsen, dadurch gekennzeichnet, daß es auf seiner Oberfläche und/oder in seinem Inneren mindestens eine photochrome Verbindung gemäß Anspruch 1 oder 2 in Mengen enthält, die dazu ausreichen, daß das Material bei Einwirkung von ultravioletter Strahlung die Farbe wechselt.

8. Transparentes festes Material nach Anspruch 7, dadurch gekennzeichnet, daS es 0,01 bis 20 Gew.-% photochrome Verbindungen enthält.

9. Zusammensetzung oder transparentes festes Material nach einem der Ansprüche 4 bis 8, dadurch gekennzeichnet, daß die photochrome Verbindung gemäß Anspruch 1 oder 2 zusammen mit anderen photochromen Verbindungen, die verschiedene Färbungen ergeben, verwendet wird.

10. Transferlack, dadurch gekennzeichnet, daß er mindestens eine Verbindung gemäß Anspruch 1 oder 2 enthält.
